# DEMANDE DE BREVET EUROPEEN

(11) **EP 4 566 662 A1**
(43) Date de publication de la demande: **11.06.2025**
(21) Numéro de dépôt: 24213565.5
(22) Date de dépôt: 18.11.2024
(51) Int. Cl.: A61N 5/06, A61N 5/067, H01L 21/00, H01S 5/183, H01S 5/40, H01S 5/343

(54) **SONDE MÉDICALE POUR OPTOGÉNÉTIQUE AVEC DES VCSELS**

(30) Priorité: 08.12.2023 FR 2313839
(71) Demandeur: Commissariat à l'Energie Atomique et aux Energies Alternatives, 75015 Paris (FR)
(72) Inventeur: GARREAU, Alexandre, 91300 MASSY (FR); SAUTER-STARACE, Fabien, 38054 GRENOBLE CEDEX 09 (FR); PARET, Jean-François, 91300 MASSY (FR); MARTIN, Marie-Blandine, 91767 PALAISEAU CEDEX (FR)
(74) Mandataire: Atout PI Laplace

(57) **Abrégé**

L'invention concerne une sonde médicale (MP) pour optogénétique comprenant :
- un substrat souple en matériau bidimensionnel conducteur (M2DS),
- une pluralité de microlasers à semi-conducteur III-V à cavité verticale et émission par la surface (µVL), dénommés lasers élémentaires, comprenant :
∘ une couche active (AL) disposée entre une couche réflectrice inférieure (BBR) et une couche réflectrice supérieure (TBR),
∘ un contact semiconducteur inférieur (BSCC) disposé entre la couche réflectrice inférieure et le substrat et un contact métallique inférieur (BMC) disposé sur le substrat et connecté audit contact semiconducteur inférieur (BSCC) via ledit substrat (M2DS),
∘ un contact semiconducteur supérieur (TSCC) disposé sur la couche réflectrice supérieure (TBR), et un contact métallique supérieur (TMC) connecté audit contact semiconducteur supérieur,
∘ les contacts métalliques inférieurs des lasers élémentaires étant destinés à être connectés électriquement à un potentiel commun,

- une couche d'encapsulation biocompatible.

## Description

### DOMAINE DE L'INVENTION

La présente invention concerne le domaine de l'optogénétique, et plus particulièrement celui des sondes médicales pour sa mise en oeuvre.

### ETAT DE LA TECHNIQUE

L'optogénétique est une technique qui consiste à modifier génétiquement des neurones ou des nerfs, tel que le nerf auditif, afin qu'ils deviennent sensibles à la lumière grâce à l'expression d'une protéine : l'opsine.

Un exemple d'application est l'amélioration de l'audition.

Une solution classique est de réaliser des implants cochléaires (IC), considérés comme la neuroprothèse la plus performante qui permet aux patients souffrant d'une perte auditive neurosensorielle de comprendre la parole. En stimulant électriquement le nerf auditif, les implants cochléaires constituent une interface qui reconnecte le cerveau du patient à la scène auditive. Cependant, comme il est difficile de concentrer le courant électrique dans des environnements conducteurs tels que la cochlée, la précision du codage électrique du son et la qualité de l'audition artificielle sont limitées.

Le principe de l'audition est brièvement rappelé. L'onde sonore représente une alternance de zones de haute et de basse pression. La membrane tympanique vibre en réponse à une onde sonore. Cette vibration est amplifiée lorsqu'elle traverse les osselets (malléus, incus et étrier).

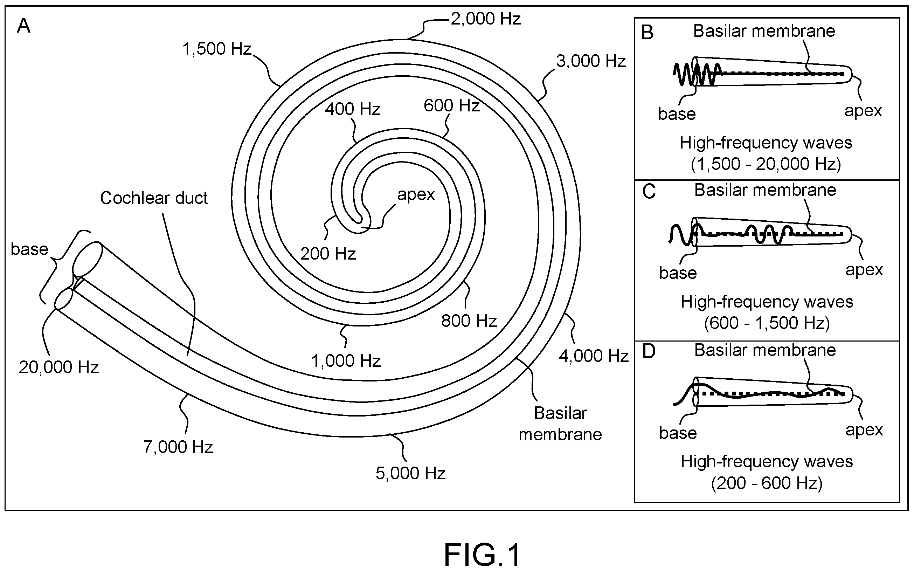

La vibration amplifiée est captée par la fenêtre ovale, ce qui provoque des ondes de pression dans le liquide de la scala vestibuli et de la scala tympani de la cochlée, dont la longueur est d'environ 34 mm. La fonction de la cochlée est de cartographier les sons de différentes fréquences sur des positions caractéristiques correspondantes de la membrane basilaire, tel qu'illustré figure 1.

Les stéréocils sont des protubérances à base d'actine sur les cellules sensorielles auditives et vestibulaires qui sont nécessaires à l'audition. Ils convertissent la force physique du son en un signal électrique par une transduction mécano-électrique. Une homéostasie défectueuse des stéréocils est l'une des principales causes de la surdité progressive liée à l'âge.

La stimulation optogénétique de la cochlée est une approche alternative intéressante pour la restauration de l'audition. L'optogénétique cochléaire promet une meilleure sélectivité spectrale du codage sonore artificiel. Elle repose sur l'utilisation d'opsines injectées dans la branche cochléaire du nerf crânien VIII, correspondant au nerf vestibulocochléaire, et d'un dispositif optique stimulant les opsines. Les opsines sont des protéines sensibles à la lumière qui assurent la conversion d'un photon en un signal électrochimique.

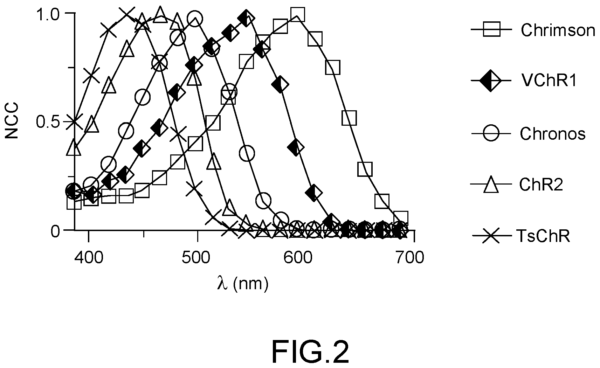

La courbe de sensibilité de cette opsine en fonction de la longueur d'onde de l'excitation lumineuse est donnée par exemple par la publication de Klapoetke et al « Independent optical excitation of distinct neural populations » Nat. Methods 11, 338-346 (2014). La charge cumulative normalisée ou NCC (pour « Normalized cumulative charge ») en fonction de la longueur d'onde λ, qui traduit la sensibilité de l'opsine, est illustré figure 2 pour différents types d'opsine : Chrimson ; VChR1 ; Chronos ; ChR2 ; TsChR. Par exemple pour Chrimson et ses variantes, la sensibilité maximale se situe à une longueur d'onde de l'ordre de 594 nm, et pour TsChR la sensibilité maximale est à environ 440 nm.

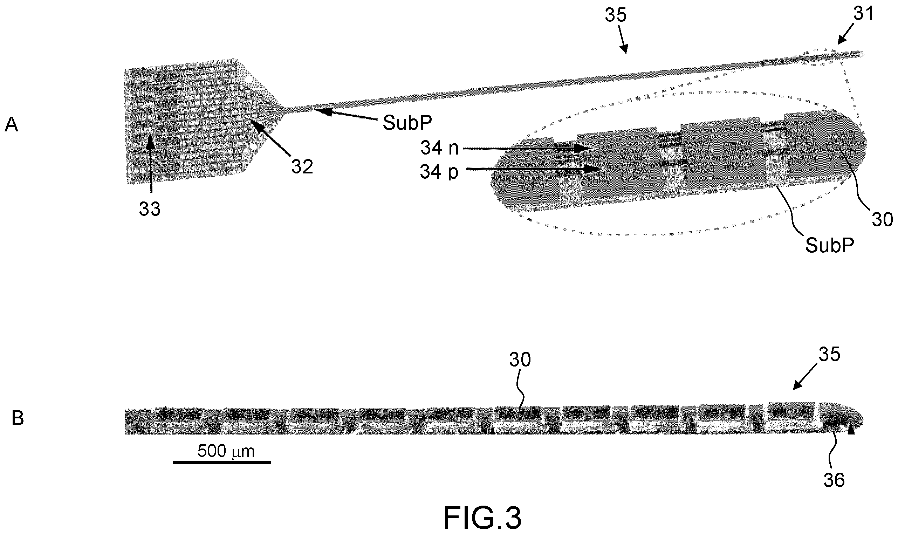

La publication de Keppeler et al « Multichannel optogenetic stimulation of the auditory pathway using microfabricated LED cochlear implants in rodents" Science Translational Medecine 12 (2020) décrit un implant cochléaire optique 35 (optogénétique) constitué d'un réseau flexible de micro-LED à base de nitrure de gallium (GaN) utilisé pour une application dans un modèle de souris, et illustré figure 3. Les LEDs 30 de 50x50 µm² sont disposées sur un substrat SubP en polyimide, à une de ses extrémités 31, selon un pas de 350 µm. La partie sur laquelle sont disposées les LED 30 est destinée à être insérée dans le canal cochléaire, le substrat doit donc être très souple. Les LEDs sont alimentées via des pistes électriques connectées à l'autre extrémité à des fils 32 reliés à une interface de connexion 33. Chaque LED est alimentée via deux contacts, un contact n et un contact p, connectés respectivement aux pistes électriques 34n et 34p, le contact n étant commun à toutes les LEDs selon une piste unique 34p. L'ensemble substrat/LEDs est encapsulé dans une couche de silicone 36 (voir B de la figure 3).

La fabrication de dispositifs de 15 µm d'épaisseur et très flexibles en polyimide est rendue possible par un procédé de transfert au laser des LED GaN sur saphir à une plaquette de support en polyimide sur silicium. Avec ce procédé de transfert les LEDS sont positionnées une à une sur le substrat, ce qui rend la fabrication de l'implant longue et coûteuse, et limite le nombre d'émetteurs du dispositif. De plus la grande surface des GaN-LEDs induit un profil de faisceau important, ce qui limite le nombre d'émetteurs optiques en raison d'un risque d'interaction entre deux émetteurs optiques voisins. En outre, la grande surface des GaN-LEDs augmente la température à l'intérieur de la cochlée, ce qui oblige à trouver des solutions pour évacuer la chaleur.

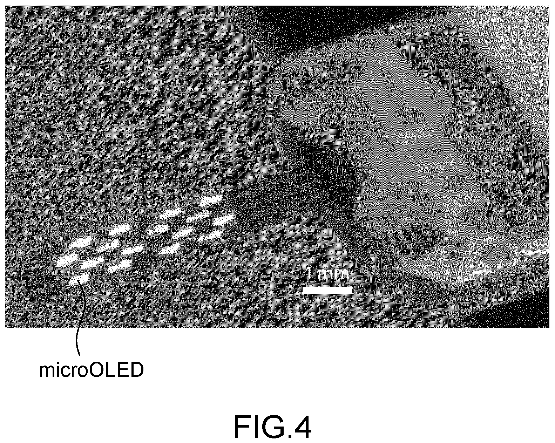

Une solution alternative à base de microOLED est décrit dans la publication de Sheppard et al « Optogenetic stimulation probes with single-neuro resolution based on organic LED monolithically integrated on CMOS » Nature Electronics, Vol. 6 p 669-679 (2023). Le dispositif est illustré figure 4. La conception et la caractérisation d'une tige de silicium incorporant une haute densité de sources lumineuses organiques. Ces microOLEDs de dimension 20 µm x 20 µm avec un pas de 25 µm ont l'avantage d'être traitées après le pilote électronique. Il s'agit d'un dispositif dit « above-IC » ce qui signifie que les dépôts et structurations sont réalisés après la réalisation de puce (IC), par-dessus, car la puissance optique des microOLEDs sont faibles. Bien que très peu lumineuses, il est validé qu'elles peuvent déclencher un potentiel d'action associé à l'opsine ChRmine qui est deux ordres de grandeur plus sensible que l'opsine ChrimsonR décrite par la publication de Klapoetke et al précitée, et sensible à la stimulation optique colorée en orange.

De même que précédemment, un inconvénient important de ces émetteurs est leur rendement médiocre, qui induit une transformation du courant d'alimentation en chaleur par effet joule. Or les normes médicales ne permettent pas un échauffement des tissus supérieur à 2°C, ce qui limite le nombre d'émetteurs utilisables. Les technos OLED sont en outre sensibles à l'humidité, ce qui induit l'utilisation d'un empilement de couches de type atomique « Atomic Layer Déposition » (ALD) et de parylène, pour assurer la biocompatibilité et limiter la migration d'humidité. La compatibilité de ces composants avec une utilisation chronique n'est pas acquise.

Un but de la présente invention est de remédier aux inconvénients précités en proposant une sonde médicale pour optogénétique présentant des émetteurs de petite taille et avec de meilleures performances que les implants optogénétique de l'état de la technique, et un procédé de fabrication parallélisé.

### DESCRIPTION DE L'INVENTION

La présente invention a pour objet une sonde médicale pour optogénétique comprenant :
- un substrat souple en matériau bidimensionnel conducteur,
- une pluralité de microlasers à semi-conducteur III-V à cavité verticale et émission par la surface, dénommés lasers élémentaires, les lasers élémentaires étant disposés sur ledit substrat et intégrés dans une couche isolante, les lasers élémentaires présentant une dimension maximale comprise entre 5 et 50 µm et comprenant :
   ∘ une couche active disposée entre une couche réflectrice inférieure et une couche réflectrice supérieure,
   ∘ un contact semiconducteur inférieur disposé entre la couche réflectrice inférieure et le substrat, et un contact métallique inférieur disposé sur le substrat et connecté audit contact semiconducteur inférieur via ledit substrat,
   ∘ un contact semiconducteur supérieur disposé sur la couche réflectrice supérieure et un contact métallique supérieur connecté audit contact semiconducteur supérieur,
   ∘ les contacts métalliques inférieurs des lasers élémentaires étant destinés à être connectés électriquement à un potentiel commun,
- une couche d'encapsulation biocompatible.

Selon un premier mode de réalisation le matériau bidimensionnel est le graphène.

Selon un deuxième mode de réalisation le matériau bidimensionnel est un dichalcogénure ou un trichalcogénure configuré pour être conducteur.

Selon un mode de réalisation le substrat présente une forme de ruban sur une partie duquel lesdits lasers élémentaires de ladite pluralité sont disposés en ligne, et les contacts métalliques inférieurs des lasers élémentaires sont connectés à une piste électrique inférieure commune audits lasers élémentaires de la ligne.

Selon un autre mode de réalisation lasers élémentaires de ladite pluralité sont disposés selon une matrice, les contacts semiconducteurs inférieurs des lasers élémentaires d'une ligne de la matrice étant connectés à une piste électrique inférieure commune aux lasers élémentaires de ladite ligne de la matrice, les pistes électriques inférieure associées aux lignes étant connectées entre elles.

Selon une première variante les contacts semiconducteurs inférieur et supérieur d'un laser élémentaire sont en nitrure de gallium ou en un matériau ternaire comprenant du nitrure de gallium.

Selon un mode de réalisation de la première variante précédente le matériau bidimensionnel est le graphène et le contact semiconducteur inférieur présente un axe cristallographique de croissance selon l'axe [1000].

Selon un autre mode de réalisation de la première variante le matériau bidimensionnel est un dichalcogénure choisi parmi WS₂, MoS₂, ReS₂ et le contact semiconducteur inférieur présente un axe cristallographique de croissance selon l'axe [100].

Selon une deuxième variante les contacts semiconducteurs inférieur et supérieur d'un laser élémentaire sont en arséniure de gallium ou en un matériau ternaire comprenant de l'arséniure de gallium.

Selon un mode de réalisation de la deuxième variante le matériau bidimensionnel est le graphène, et le substrat en graphène comprend des logements dans lesquels sont disposés les lasers élémentaires.

Selon un mode de réalisation la couche active comprend des multipuits quantiques ou des points quantiques.

Selon un mode de réalisation le substrat en matériau bidimensionnel et les lasers élémentaires forment une première structure, la sonde comprenant au moins une deuxième structure empilée sur la première structure, des lasers élémentaires des deux structures étant agencés de sorte que des lasers élémentaires de la deuxième structure n'occultent pas un faisceau émis par des lasers élémentaires de la première structure.

Selon un mode de réalisation les lasers élémentaires dans la deuxième structure sont configurés pour émettre une longueur d'onde différente d'une longueur d'onde d'émission de la première structure.

Selon un mode de réalisation une des deux structures est constituée de lasers élémentaires comprenant au moins une couche en nitrure de gallium ou un matériau ternaire comprenant du nitrure de gallium, et l'autre structure est constituée de lasers élémentaires comprenant au moins une couche en arséniure de gallium ou un matériau ternaire comprenant du nitrure de gallium.

Selon un autre aspect l'invention concerne un premier procédé de fabrication d'une sonde médicale pour optogénétique comprenant les étapes consistant à :
**A1** disposer d'un premier substrat initial comprenant un substrat semiconducteur, une couche isolante dite de substrat, une couche métallique et une couche de graphène disposé sur la couche métallique,
**B1** déposer sur le premier substrat initial une première couche diélectrique et une première couche de résine, structurer la première couche de résine de manière à former un premier masque présentant des premières ouvertures, les premières ouvertures présentant une dimension maximale comprise entre 5 et 50 µm,
**C1** graver la première couche diélectrique jusqu'à la couche de graphène par gravure humide, de manière à mettre à nu la couche de graphène dans les premières ouvertures,
**D1** enlever la première couche de résine,
**E1** réaliser par épitaxie, dans lesdites premières ouvertures, un empilement de matériaux semiconducteurs, l'empilement comprenant un contact semiconducteur inférieur en nitrure de gallium épitaxié sur la couche de graphène, une couche réflectrice inférieure, une couche active, une couche réflectrice supérieure et un contact semiconducteur supérieur en nitrure de gallium,
**F1** déposer une deuxième couche diélectrique, structurer une deuxième couche de résine de manière à former un deuxième masque présentant des deuxièmes ouvertures au-dessus de chaque empilement, graver la deuxième couche diélectrique et le contact semiconducteur supérieur et enlever la deuxième couche de résine,
**G1** enlever les première et deuxième couches diélectriques par gravure humide, de manière à obtenir des empilements disposés sur le premier substrat initial,
**H1** déposer un contact métallique inférieur sur la couche de graphène, déposer une couche isolante autour des empilements et déposer un contact métallique supérieur connecté avec le contact semiconducteur supérieur,
**I1** retirer le substrat semiconducteur à l'aide de la couche isolante de substrat,
**J1** retirer la couche métallique.
De plus, les empilements et les premier et deuxième contacts métalliques associés sont configurés pour former des microlasers semi-conducteur à cavité verticale et émission par la surface insérés dans la couche isolante, dénommés lasers élémentaires, la couche de graphène formant un substrat souple, les lasers élémentaires étant disposés sur ledit substrat souple. Le procédé comprend en outre une étape **K1** d'encapsulation du substrat et des lasers élémentaires avec un matériau biocompatible.

Selon un mode de réalisation, lors de l'étape **E1** la croissance du contact semiconducteur inférieur en nitrure de gallium par épitaxie sur le substrat en graphène s'opère selon une direction [1000].

Selon un mode de réalisation, à l'étape **C1** la gravure humide de la première couche diélectrique jusqu'à la couche de graphène est de type BOE pour « Buffered Oxide Etching ».

Selon un autre aspect l'invention concerne un deuxième procédé de fabrication d'une sonde médicale pour optogénétique comprenant les étapes consistant à :
**A2** disposer d'un deuxième substrat initial comprenant un substrat en arséniure de gallium, une couche isolante dite de substrat, une couche métallique et une première couche de graphène disposée sur la couche métallique,
**B2** déposer sur le deuxième substrat initial une première couche diélectrique et une première couche de résine, structurer la première couche de résine de manière à former un premier masque présentant des premières ouvertures, les premières ouvertures présentant une dimension maximale comprise entre 5 et 50 µm,
**C2** graver la première couche diélectrique jusqu'à la couche de graphène,
**D2** enlever la première couche de résine, graver la couche de graphène, graver la couche métallique et graver la couche isolante de substrat, de manière à mettre à nu le substrat en arséniure de gallium dans les premières ouvertures,
**E2** réaliser par épitaxie, dans lesdites premières ouvertures, un empilement de matériaux semiconducteurs comprenant un contact semiconducteur inférieur en arséniure de gallium épitaxié sur le substrat en arséniure de gallium, une couche réflectrice inférieure, une couche active, une couche réflectrice supérieure et un contact semiconducteur supérieur en arséniure de gallium,
**F2** déposer une deuxième couche diélectrique et une deuxième couche de résine, structurer la deuxième couche de résine de manière à former un deuxième masque présentant des deuxièmes ouvertures au-dessus de chaque empilement, graver la deuxième couche diélectrique, graver le contact semiconducteur supérieur, et enlever la deuxième couche de résine,
**G2** enlever les première et deuxième couches diélectriques par gravure humide, de manière à obtenir des empilements disposés sur le substrat en arséniure de gallium,
**H2** déposer un contact métallique inférieur sur la couche de graphène restante de chaque côté des empilements, déposer une couche isolante autour des empilements et déposer un contact métallique supérieur en contact avec le contact semiconducteur supérieur,
**I2** retirer le substrat en arséniure de gallium à l'aide de la couche isolante de substrat,
**J2** retirer la couche métallique, et déposer une deuxième couche de graphène sur la première couche de graphène et le contact semiconducteur inférieur, la première et la deuxième couche de graphène formant collectivement un substrat souple en graphène.
De plus les empilements et les premier et deuxième contacts métallique associés sont configurés pour former des microlasers semi-conducteur à cavité verticale et émission par la surface (µVL) insérés dans la couche isolante, dénommés lasers élémentaires, les lasers élémentaires étant disposés sur ledit substrat en graphène, le substrat en graphène comprenant des logements dans lesquels sont disposés les lasers élémentaires. En outre le procédé comprend une étape **K2** d'encapsulation du substrat et des lasers élémentaires avec un matériau biocompatible.

La description suivante présente plusieurs exemples de réalisation du dispositif de l'invention : ces exemples sont non limitatifs de la portée de l'invention. Ces exemples de réalisation présentent à la fois les caractéristiques essentielles de l'invention ainsi que des caractéristiques additionnelles liées aux modes de réalisation considérés.

L'invention sera mieux comprise et d'autres caractéristiques, buts et avantages de celle-ci apparaîtront au cours de la description détaillée qui va suivre et en regard des dessins annexés donnés à titre d'exemples non limitatifs et sur lesquels :
La figure 1 déjà citée illustre le schéma de principe de transmission du son par la cochlée.
La figure 2 déjà citée illustre la charge cumulative normalisée en fonction de la longueur d'onde, pour différents types d'opsine.
La figure 3 déjà citée illustre un implant cochléaire optique constitué d'un réseau flexible de micro-LED à base de nitrure de gallium pour une souris.
La figure 4 déjà citée illustre un implant constitué de microOLED sur CMOS.
La figure 5 illustre un mode de réalisation d'une sonde selon l'invention dans laquelle le substrat présente une forme de ruban.
La figure 6 illustre un mode de réalisation d'une sonde selon l'invention dans laquelle les lasers élémentaires sont disposés selon un réseau planaire en matrice.
La figure 7 illustre un mode de réalisation d'une sonde selon l'invention dans laquelle le substrat en graphène comprend des logements dans lesquels sont disposés les lasers élémentaires à base de GaAs.
La figure 8 illustre un mode de réalisation d'une sonde selon l'invention dans laquelle le substrat en matériau 2D et les lasers élémentaires forment une première structure S1, et la sonde comprend au moins une deuxième structure S2 empilée sur la première structure S1, la structure S2 présentant une architecture identique à la structure S1 (substrat + lasers élémentaires).
La figure 9 illustre les étapes **A1** à **F1** du procédé 100 selon l'invention.
La figure 10 illustre les étapes **G1** à **J1** du procédé 100 selon l'invention.
La figure 11 illustre les étapes **A2** à **F2** du procédé 100 selon l'invention.
La figure 12 illustre les étapes **G2** à **J2** du procédé 100 selon l'invention.

### DESCRIPTION DETAILLEE DE L'INVENTION

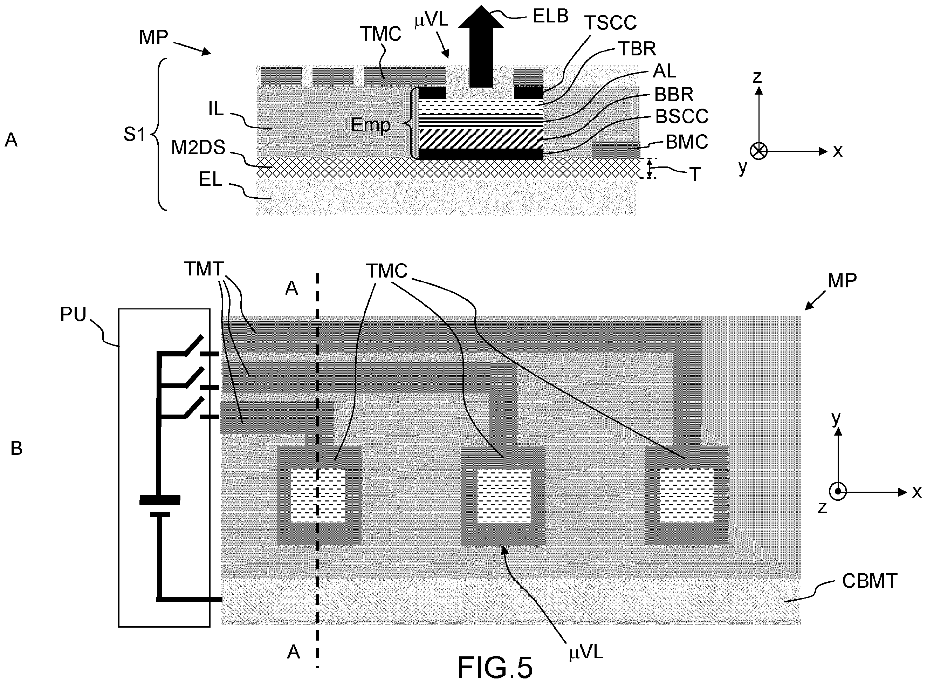

Un mode de réalisation de la sonde médicale MP selon l'invention, adaptée pour la réalisation d'un implant cochléaire avec un substrat en forme ruban et des émetteurs en ligne, est illustré figure 5. La partie B est une vue du dessus dans un plan XY de la sonde selon l'invention et la partie A est une vue de profil dans un plan XZ (coupe selon AA).

La conception de l'implant cochléaire est facilitée du fait que la topologie de la cochlée est bien connue avec sa distribution de cellules ciliées spécifiques aux fréquences sonores. Après avoir enregistré et analysé la fréquence d'un son émis, un émetteur de la sonde optique, dont la localisation dans la cochlée correspond à cette fréquence, est mis en marche. Il permet de stimuler la branche cochléaire du nerf crânien VIII dans laquelle des opsines ont été injectées et de générer ainsi une perception sonore artificielle.

Cependant le principe de stimulation d'un neurone ou d'un nerf par un dispositif optique via l'opsine est applicable à toute autre application de l'optogénétique, en modifiant la conception du dispositif. L'invention est ainsi applicable à d'autres types de sondes pour optogénétique ayant des géométries d'agencement des émetteurs différents, par exemple un réseau planaire, une tige, un tube obtenu à partir d'un réseau planaire (grâce au substrat souple) ... La sonde selon l'invention trouve à s'appliquer par exemple pour une intégration dans le cortex visuel pour la restauration de la vue ou dans le cortex moteur pour la compensation des handicaps moteurs.

La sonde médicale MP pour optogénétique selon l'invention comprend un substrat M2DS souple en matériau bidimensionnel, dit 2D, configuré pour être conducteur.

Les matériaux 2D présentent une structure planaire et sont composés de une à quelques monocouches L, chaque monocouche comprenant quelques plans atomiques (typiquement 1 à 20), le nombre de plans atomiques étant fonction de la structure atomique. Les liaisons chimiques à l'intérieur d'une monocouche sont covalentes. Un matériau bidimensionnel peut être conducteur ou semiconducteur, typiquement en fonction du nombre de monocouches empilées.

Selon un mode de réalisation le matériau bidimensionnel est le graphène. Le graphène est naturellement conducteur et composé d'un seul atome, le carbone, et la monocouche ne comprend qu'un plan atomique, elle est plane et constituée d'atomes de carbone disposés en forme de réseau hexagonal. Le graphène est souple et l'épaisseur T d'un tel substrat est typiquement comprise entre 0.3 et 20 nm en fonction du nombre de monocouches empilées. Les avantages du graphène sont que les procédés associés sont matures et qu'il est non toxique. Il présente également un bon accord de maille avec certains semiconducteurs III-V (voir plus loin).

Selon un autre mode de réalisation le matériau bidimensionnel est un dichalcogénure ou un trichalcogénure configuré pour être conducteur, présentant une épaisseur comprise entre 0.3 et 20 nm.

La sonde MP comprend également une pluralité de microlasers à semi-conducteur III-V à cavité verticale et émission par la surface µVL, dénommés lasers élémentaires, et classiquement appelés VCSEL pour Vertical Cavity Surface Emitting Laser. Les lasers élémentaires µVL sont disposés sur le substrat M2DS et intégrés dans une couche isolante IL. Les lasers élémentaires présentant une dimension maximale comprise entre 5 et 50 µm. On entend par dimension maximale la plus grande dimension latérale du laser. Typiquement les lasers ont une épaisseur (hauteur) de l'ordre de 0.5 à 5 µm.

Un laser élémentaire de la sonde selon l'invention présente une structure de couches classique et comprend une couche active AL disposée entre une couche réflectrice inférieure BBR et une couche réflectrice supérieure TBR. Il comprend également :
- un contact semiconducteur inférieur BSCC disposé entre la couche réflectrice inférieure et le substrat et un contact métallique inférieur BMC,
- un contact semiconducteur supérieur TSCC disposé sur la couche réflectrice supérieure TBR et un contact métallique supérieur TMC connecté au contact semiconducteur supérieur.

Dans la configuration de l'invention le contact métallique inférieur est disposé sur le substrat M2DS et connecté électriquement au contact semiconducteur inférieur BSCC via le substrat qui est conducteur. Il convient cependant pour la connectivité électrique soit suffisante que le contact métallique inférieur ne soit pas trop éloigné du contact semiconducteur inférieur.

En outre les contacts métalliques inférieurs des lasers élémentaires sont destinés à être connectés électriquement à un potentiel commun, typiquement un potentiel de référence, une masse.

De manière classique pour une sonde présentant plusieurs émetteurs (voir par exemple figure 3) les contacts métalliques inférieurs et supérieurs sont connectés à des pistes électriques associées, typiquement métalliques, qui transportent le signal électrique de commande des émetteurs.

La sonde médicale MP selon l'invention est destinée à être connectée électriquement à une unité d'alimentation et de commande PU, connectée aux pistes.

Pour les lasers élémentaires de type VCSEL selon l'invention les contacts métalliques inférieurs sont connectés électriquement entre eux via le substrat conducteur, et sont destinés à être connectés au potentiel commun typiquement via des pistes connectées, à l'extrémité de la sonde, à l'unité d'alimentation et de commande.

Préférentiellement les contacts semiconducteurs inférieurs sont connectés à une ou plusieurs pistes électriques inférieures CMT (fonction de l'agencement des lasers élémentaires) disposées sur le substrat.

Malgré le caractère conducteur du substrat les contacts métalliques inférieurs et les pistes électriques inférieures sont nécessaires pour transporter le courant circulant dans les lasers élémentaires. Il existe typiquement un facteur d'au moins 4 à 5 entre la conductivité d'un métal (or, cuivre) et celle d'un matériau 2D.

Les contacts métalliques supérieurs sont connectés à des pistes électriques supérieures TMT. Pour chaque laser le contact électrique supérieur TMC est connecté à une piste supérieure TMT associée au laser qui transporte le signal de commande du laser élémentaire.

Selon le mode de réalisation de la figure 5 le substrat est en forme de ruban, sur une partie duquel sont disposés des lasers en ligne. Les contacts métalliques inférieurs de la pluralité de lasers sont connectés à une piste électrique inférieure CBMT commune aux lasers élémentaires de la ligne, destinée à être connectée à la masse électrique du dispositif. Cela permet de diviser par deux le nombre de contacts électriques. Cette géométrie est bien adaptée à un implant cochléaire.

La sonde MP comprend également une couche d'encapsulation biocompatible, électriquement isolante, transparente et stable à long terme, par exemple une silicone de grade médical, qui entoure au moins la partie de la sonde destinée à être insérée dans le corps.

L'originalité de l'invention consiste en la réalisation d'une sonde médicale pour optogénétique comportant des VCSELs de petite dimension sur un substrat graphène qui est fin et souple, cette structure présentant de nombreux avantages par rapport aux sondes existantes.

Tout d'abord la petite dimension des lasers élémentaires et la nature très directive du faisceau lumineux émis ELB permet un agencement très dense des émetteurs sur le substrat en matériau 2D, avec la limite de pouvoir passer le câblage électrique le cas échéant (typiquement les pistes ont une largeur de quelques microns. Pour des lasers en ligne tel que sur la figure 5 partie B, les lasers peuvent être très rapprochés car il n'y a pas de pistes entre deux lasers. Un agencement dense permet l'adressage des cellules cibles avec une très bonne résolution spatiale.

En outre les VCSEL ont un bien meilleur rendement quantique que les LEDs (plus d'électrons transformés en photons), ce qui induit une génération de chaleur par effet Joule bien moindre. Cela est très important pour les sondes médicales puisque l'échauffement admis du dispositif est très limité. Il n'est pas donc pas nécessaire de prévoir un dispositif additionnel d'évacuation de la chaleur, ce qui permet également la densification des émetteurs sans émission de chaleur excessive.

Enfin le substrat en matériau 2D apporte une grande souplesse au dispositif, lui permettant de s'adapter à différents environnements du corps humain.

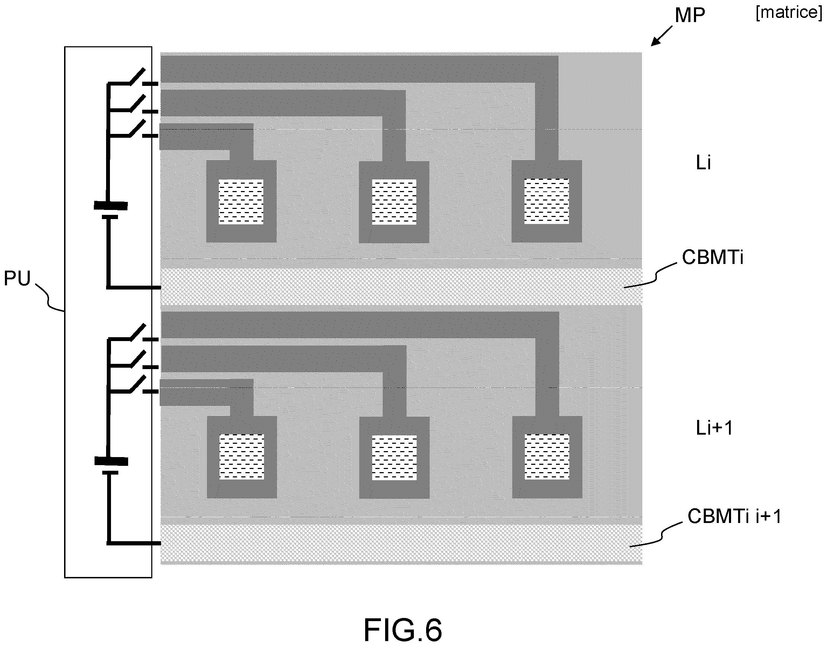

Selon un mode de réalisation les lasers élémentaires sont disposés selon un réseau planaire en matrice tel qu'illustré figure 6. Préférentiellement aux lasers élémentaires d'une ligne Li (indice i) de la matrice sont connectés à une la piste électrique inférieure partagée associée CBMTi

Le laser élémentaire µVL est un laser à cavité verticale à émission par la surface basé sur un empilement de couches semi-conductrices III-V. Préférentiellement il comprend un contact III-V dopé p, un réflecteur de Bragg III-V dopé p, la couche active AL, un réflecteur de Bragg III-V dopé n et un contact III-V dopé p.

Selon un mode de réalisation la couche active AL du laser contient des puits quantiques multiples MQW basés sur des semi-conducteurs III-V, dont la structure est un empilement alternatif de puits et de barrières composés de semi-conducteurs III-V. Selon un autre mode de réalisation la couche active du laser contient des points quantiques QD qui sont basés sur des semi-conducteurs III-V.

Selon une première variante de la sonde selon l'invention, les contacts semiconducteurs inférieur BSCC et supérieur TSCC d'un laser élémentaire sont en nitrure de gallium GaN ou un matériau ternaire comprenant du nitrure de gallium. Cette famille de VCSEL à base de GaN émet un faisceau lumineux ELB présentant une longueur d'onde dans une gamme allant du bleu au vert.

Selon une deuxième variante de la sonde selon l'invention, les contacts semiconducteurs inférieur BSCC et supérieur TSCC d'un laser élémentaire sont en arséniure de gallium (GaAs) ou un matériau ternaire comprenant de l'arséniure de gallium. Cette famille de VCSEL à base de GaAs émet un faisceau lumineux ELB présentant une longueur d'onde dans une gamme allant de l'orange au rouge voir à l'infrarouge en fonction de la nature de la couche active.

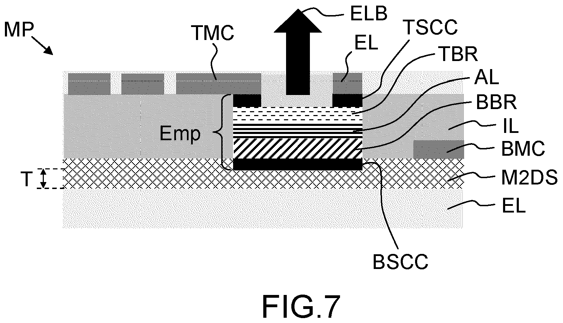

Comme il sera vu plus loin des étapes du procédé de fabrication des lasers élémentaires sur un substrat en matériau 2D qui est du graphène diffèrent pour les deux familles de composant laser semiconducteur III-V. Cela a pour conséquence que les lasers µVL à base de GaAs sont bien agencés sur le substrat en graphène mais celui-ci comprend des logements dans lesquels sont disposés les lasers élémentaires, tel qu'illustré figure 7.

Les deux familles de VCSEL permettent de cibler deux gammes de longueur d'onde différentes.

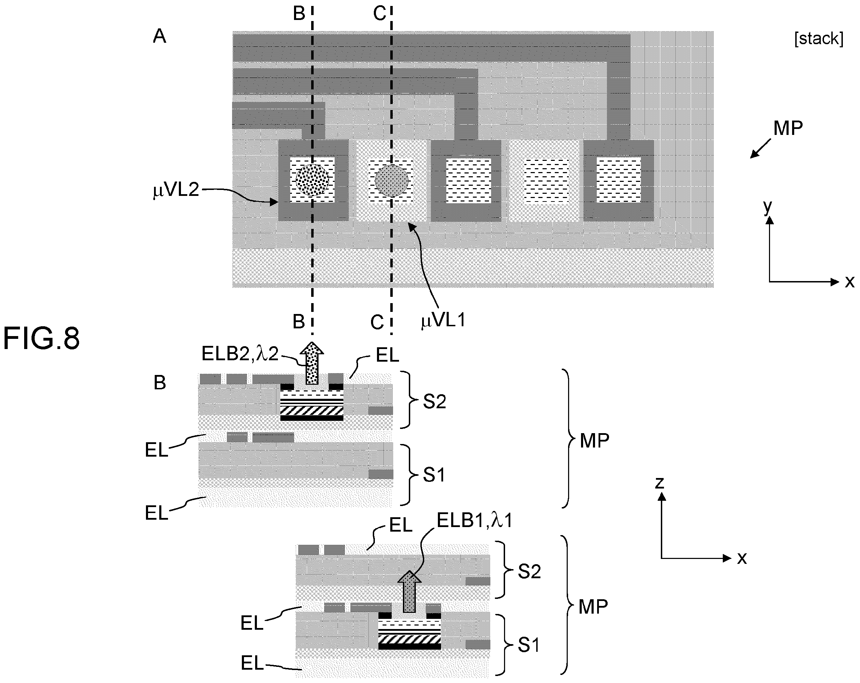

Selon un mode de réalisation illustré figure 8 on considère que le substrat en matériau 2D et les lasers élémentaires forment une première structure S1, et la sonde MP comprend au moins une deuxième structure S2 empilée sur la première structure S1, la structure S2 présentant une architecture identique à la structure S1 (substrat + lasers élémentaires). C'est alors l'ensemble S1+S2 qui est enrobé dans une couche d'encapsulation. Préférentiellement des lasers élémentaires des deux structures sont agencés de sorte que des lasers élémentaires µVL2 de la deuxième structure n'occultent pas un faisceau émis ELB1 par des lasers élémentaires µVL1 de la première structure tel qu'illustré sur la partie A de la figure 8 (vue de dessus de la sonde). La partie B illustre une vue de profil selon les coupes BB et CC.

Préférentiellement les lasers élémentaires dans la deuxième structure sont configurés pour émettre une longueur d'onde λ2 différente d'une longueur d'onde d'émission λ1 de la première structure. Cela permet de disposer d'une sonde qui peut être localisée à proximité des cellules d'intérêt et émettre tantôt une couleur tantôt une autre. Une telle sonde permet, en combinaison avec des cellules intégrant une opsine excitatrice (par exemple à λ1) et une opsine inhibitrice (par exemple à λ2), d'exciter et d'inhiber les canaux des cellules sur commande en allumant et éteignant les émetteurs associés de la sonde à double niveau.

Selon un mode de réalisation de la sonde double niveau de la figure 8, une des deux structures est constituée de lasers élémentaires comprenant au moins une couche en nitrure de gallium (famille de composant à base de GaN) et l'autre structure est constituée de lasers élémentaires comprenant au moins une couche en arséniure de gallium (famille de composant à base de GaAs).

Selon un mode de réalisation d'un émetteur selon la première variante, le contact semiconducteur inférieur BSCC est en nitrure de gallium dopé n et le contact semiconducteur supérieur TSCC est en nitrure de gallium dopé p. Il est possible d'inverser les dopages.

Les couches réflectrices inférieure et supérieure BBR et TBR sont par exemple une alternance de couches en AlGaN et GaN, et la couche active AL comprend des multipuits quantiques en InGaN séparés par des barrières en nitrure de gallium.

Typiquement le contact semiconducteur inférieur BSCC en nitrure de gallium présente une structure cristallographique de Wurtzite.

Selon un mode de réalisation d'une sonde selon l'invention avec des émetteurs selon la première variante, le matériau bidimensionnel est le graphène et le contact semiconducteur inférieur BSCC présente un axe cristallographique de croissance selon l'axe [1000]. Pour la réalisation d'un tel émetteur sur graphène, l'accord de maille entre GaN et le graphène favorise une telle structure.

De manière classique lorsque le contact semi-conducteur inférieur est selon l'axe [1000] et que tous les matériaux de l'empilement formant le VCSEL sont à base de GaN, ces matériaux présentent tous un axe de croissance selon [1000].

Selon un autre mode de réalisation d'un émetteur selon la première variante le matériau bidimensionnel est un dichalcogénure ou un trichalcogénure. Préférentiellement le matériau est un dichalcogénure choisi parmi WS₂, MoS₂, ReS₂ et le contact semiconducteur inférieur présente un axe cristallographique de croissance selon l'axe [100]. Les matériaux précités présentent l'avantage d'avoir une structure de maille atomique proche de celle du GaN et la structure du GaN selon l'axe [100] est la plus adaptée à la croissance de GaN sur ce type de matériau.

Selon un mode de réalisation d'un émetteur selon la deuxième variante, le contact semiconducteur inférieur BSCC et le contact semiconducteur supérieur TSCC sont en GaAs ou en un matériau ternaire comprenant du GaAs.

Préférentiellement un des contacts semi-conducteur est dopé n et l'autre est dopé p.

Par exemple la couche réflectrice inférieure BBR est une alternance de deux couches en AIGaAs et AlAs, la couche active AL comprend des multipuits quantiques en GaInP séparés par des barrières en AIGalnP et la couche réflectrice supérieure TBR est une alternance de deux couches en AIGaAs de composition différente.

Selon un autre aspect l'invention concerne un procédé 100 de fabrication d'une sonde médicale pour optogénétique avec des lasers élémentaires réalisés à base de GaN (au moins le contact semi-conducteur inférieur) sur un substrat en matériau 2D de type graphène. Le graphène présente l'avantage d'avoir un procédé de mise en oeuvre mature et d'être non toxique. Il présente en outre l'avantage d'avoir une structure de maille accordée avec celle du GaN.

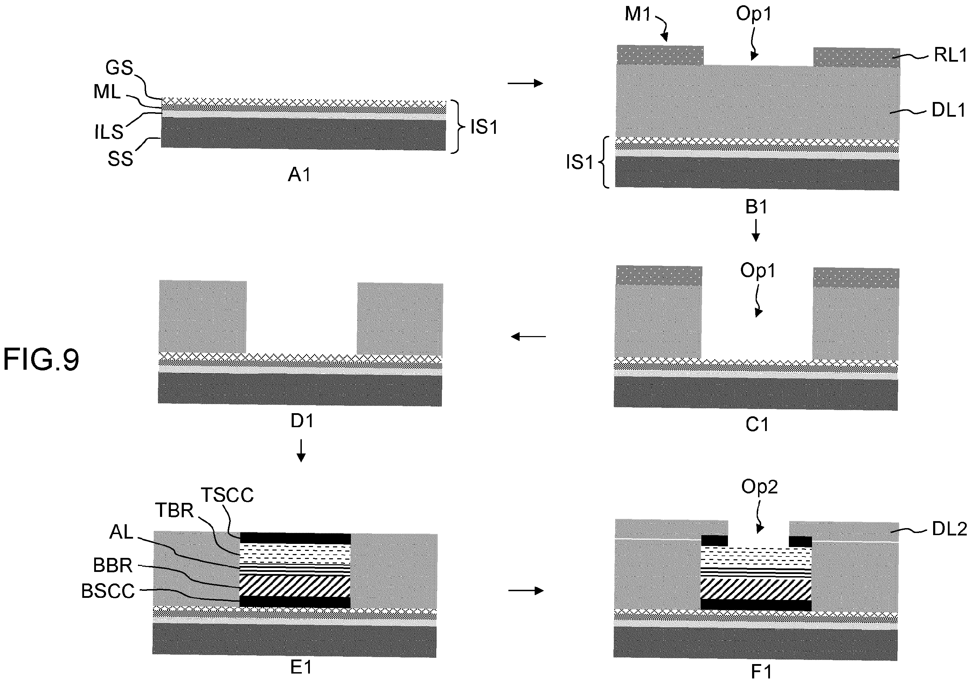

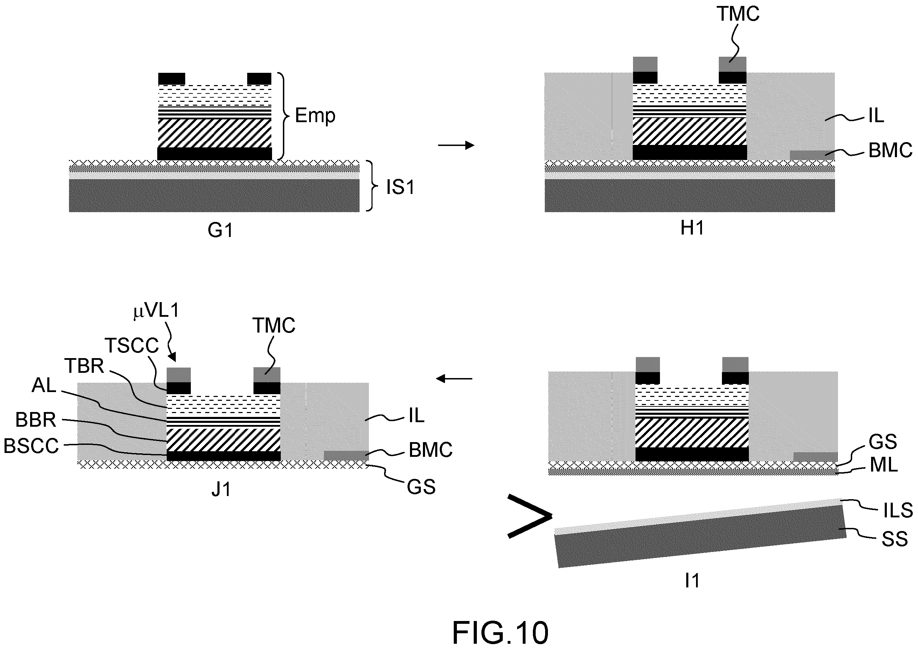

Le procédé 100 selon l'invention est illustré figure 9 pour les étapes **A1** à **F1** et figure 10 pour les étapes **G1** à **J1.**

Il comprend une première étape **A1** consistant à disposer d'un premier substrat initial IS1 comprenant un substrat semiconducteur SS, tel que du silicium, une couche isolante dite de substrat ILS, typiquement diélectrique, une couche métallique ML, par exemple en cuivre ou en nickel et une couche de graphène GS disposée sur la couche métallique. Le graphène n'est pas soluble dans ces métaux. Typiquement la couche de graphène a crû sur la couche métallique par une technique de dépôt par vapeur chimique ou CVD (pour « Chemical Vapor Déposition ») en utilisant du CH₄-H₂.

Ensuite dans une étape **B1** on dépose sur le premier substrat initial IS1 une première couche diélectrique (DL1), typiquement du SiO2 ou du Si3N4, et une première couche de résine RL1, et on structure typiquement par photolithographie la première couche de résine RL1 de manière à former un premier masque M1 présentant des premières ouvertures Op1. Les premières ouvertures présentent une dimension maximale comprise entre 5 et 50 µm.

Dans une étape **C1** on grave la première couche diélectrique DL1 jusqu'à la couche de graphène par gravure humide, de manière à mettre à nu la couche de graphène dans les premières ouvertures. Ici on s'arrête sur une couche de matériau 2D d'épaisseur atomique, et il faut conserver l'intégrité de la couche de graphène. On ne peut pas utiliser une gravure sèche de type ionique (ou RIE pour « Reactive Ion Etching ») par exemple à base de chlore, qui est forcément en partie mécanique et risquerait d'endommager le graphène. Seule une gravure humide est utilisable pour cette étape. Préférentiellement selon un mode de réalisation, on utilise une gravure de type acide fluorhydrique (HF) tamponnée telle que le BOE pour « Buffered Oxide Etching » car le graphène résiste à ce type de gravure.

Dans une étape **D1** on enlève la première couche de résine, par exemple par attaque chimique avec du 2-propanol.

On dispose alors d'emplacements dans lesquels on fait croître les VCSEL. Ainsi dans une étape **E1** on réalise par épitaxie, typiquement par MOCVD (pour « MetalOrganic Chemical Vapor Déposition » ) ou GSMBE (pour « Gas Source Molecular Beam Epitaxy ») dans les premières ouvertures Op1, un empilement de matériaux semiconducteurs. L'empilement Emp comprend un contact semiconducteur inférieur BSCC en nitrure de gallium (ou un ternaire à base de GaN) épitaxié sur la couche de graphène, une couche réflectrice inférieure BBR, une couche active AL, une couche réflectrice supérieure TBR et un contact semiconducteur supérieur TSCC en nitrure de gallium (ou un ternaire à base de GaN).

Selon un mode de réalisation préféré l'épitaxie du GaN sur le graphène s'opère selon l'axe cristallographique [1000], car il y a un bon accord de maille. Préférentiellement le contact semiconducteur inférieur BSCC présente une structure cristallographique de Wurtzite.

Ainsi dans les endroits où le graphène est à nu on fait croître des VCSEL, tous isolés les uns des autres, avec une masse commune. Grâce à cette technologie dite SAG (pour Selective Area Growth) le semiconducteur pousse sur le graphène et pas ailleurs, ce qui permet de définir la géométrie du laser sans gravure chlorée (juste la gravure de la couche diélectrique et du contact pour faire sortir la lumière).

Dans une étape **F1** on déposer une deuxième couche diélectrique DL2 (par exemple en SiO₂ ou Si₃N₄) et on structure, typiquement par photolithographie, une deuxième couche de résine RL2 de manière à former un deuxième masque M2 présentant des deuxièmes ouvertures Op2 au-dessus de chaque empilement. Puis on grave la deuxième couche diélectrique DL2, typiquement par RIE au fluor, et on grave le contact semiconducteur supérieur, typiquement par gravure RIE au chlore. Enfin on enlève la deuxième couche de résine RL2. La surface d'émission du VCSEL est à nu pour le passage de la lumière émise. La couche de graphène est protégée par la couche DL2.

A l'étape **G1** on enlève les première et deuxième couches diélectriques DL1 et DL2 par gravure humide, de manière à obtenir des empilements disposés sur le premier substrat initial. La gravure est ici humide pour les mêmes raisons que précédemment, soit pour ne pas dégrader la couche de graphène.

On dépose ensuite à l'étape **H1** un contact métallique inférieur BMC sur la couche de graphène qui est donc relié au contact semiconducteur inférieur via la couche conductrice de graphène et on déposer une couche isolante IL autour des empilements. On dépose également un contact métallique supérieur TMC connecté avec le contact semiconducteur supérieur TSCC, et de sorte que l'émission par la surface supérieure du VCSEL puisse se faire. A ce stade les lasers élémentaires sont terminés. C'est également à ce stade que l'on réalise la ou les pistes inférieures CBMT et les pistes supérieures TMT pour la connectique.

Dans une étape **I1** on retire le substrat semiconducteur SS à l'aide de la couche isolante de substrat ILS, préférentiellement par clivage mécanique. La présence de la couche isolante de substrat ILS aide au découplage entre la couche de graphène et la couche métallique ML.

Puis dans une étape **J1 on** retire la couche métallique ML, par exemple par attaque chimique à base de FeCl₃ lorsque la couche ML est en cuivre.

Les empilements et les premier et deuxième contacts métallique associés sont configurés pour former des microlasers semi-conducteur à cavité verticale et émission par la surface µVL insérés dans la couche isolante, dénommés lasers élémentaires. La couche de graphène forme un substrat souple GS, les lasers élémentaires étant disposés sur ce substrat souple.

Le procédé 100 comprenant en outre une étape **K1** d'encapsulation du substrat et des lasers élémentaires avec un matériau biocompatible.

Le procédé 100 selon l'invention permet ainsi de réaliser en parallèle un grand nombre de lasers élémentaires sur un substrat souple en graphène, en une fois et sans procédé de transfert.

Les lasers élémentaires sont tous à la même masse du fait de la conductivité du graphène.

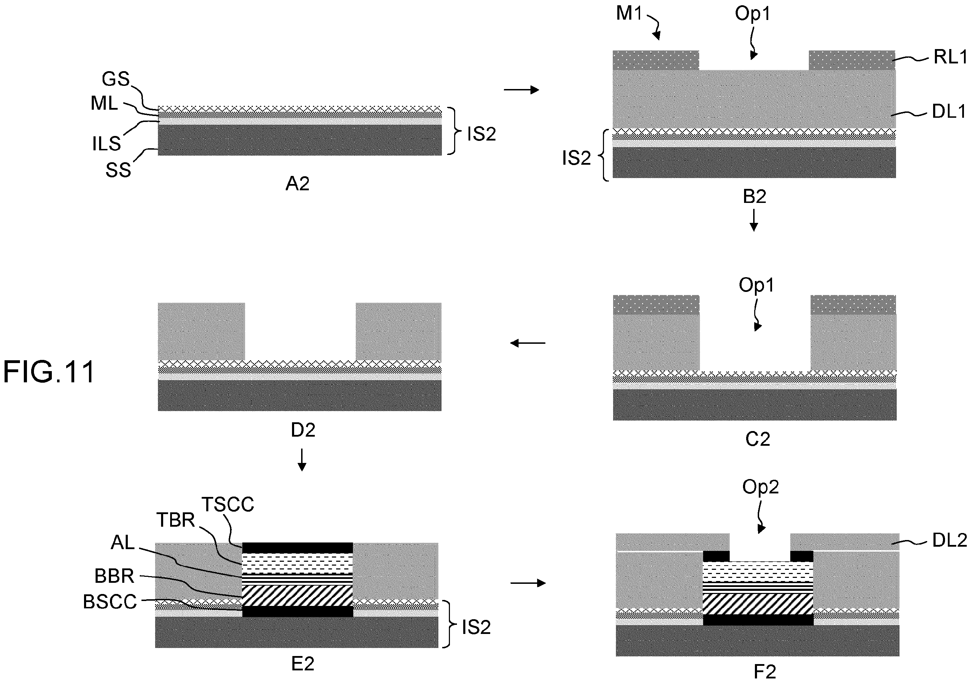

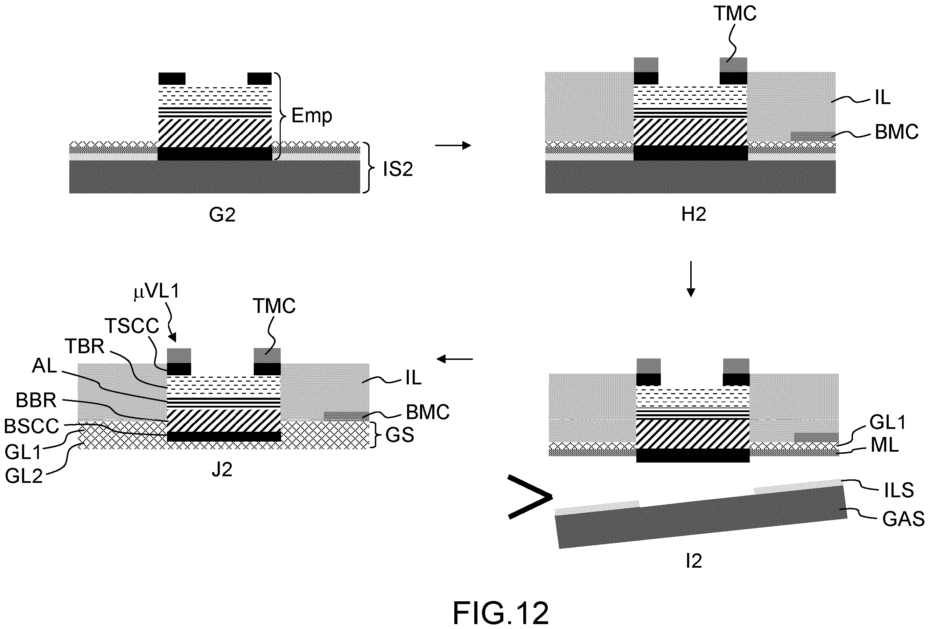

Selon un autre aspect l'invention concerne un procédé 200 de fabrication d'une sonde médicale pour optogénétique avec des lasers élémentaires réalisés à base de GaAs sur un substrat en matériau 2D de type graphène. Le procédé 200 selon l'invention est illustré figure 11 pour les étapes **A2** à **E2** et figure 12 pour les étapes **G2** à **J2.**

Le procédé 200 présente certaines étapes différentes du procédé 100 car le GaAS n'est pas accordé en maille avec les matériaux 2D tels que le graphène ou des dichalcogénures.

Dans une première étape **A2** on dispose d'un deuxième substrat initial IS2 comprenant un substrat en arseniure de gallium GAS, une couche isolante dite de substrat ILS, une couche métallique ML (par exemple en cuivre ou en nickel) et une première couche de graphène GL1 disposée sur la couche métallique.

Dans une étape **B2** déposer sur le deuxième substrat initial une première couche diélectrique DL1 et une première couche de résine RL1, on structure la première couche de résine de manière à former un premier masque M1 présentant des premières ouvertures Op1. Les premières ouvertures présentent une dimension maximale comprise entre 5 et 50 µm.

Dans une étape **C2** on grave la première couche diélectrique jusqu'à la couche de graphène. La gravure ici peut être sèche ou humide, car on va enlever la couche de graphène GL1.

Dans une étape **D2** on enlève la première couche de résine, on grave la couche de graphène, par exemple avec une gravure RIE au chlore, on graver la couche métallique, typiquement par IBE (pour « Ion Beam Etch »). On grave également la couche isolante de substrat, par exemple avec une gravure RIE au fluore de manière à mettre à nu le substrat en GaAs dans les premières ouverturesOp1.

Ainsi dans les endroits où le substrat GAS est à nu on fait croître des VCSEL, tous isolés les uns des autres, typiquement avec une masse commune (voir plus loin). Le matériau GaAs ne pousse pas sur le graphène car il n'est pas adapté à la maille du graphène. Dans le procédé 200 qui est toujours de type SAG dans les ouvertures Op1, le graphène devient un masque de croissance.

Dans une étape **E2** on réalise par épitaxie, typiquement MOCVD ou GSMBE, dans les premières ouvertures Op1, un empilement de matériaux semiconducteurs comprenant un contact semiconducteur inférieur BSCC en arséniure de gallium (ou en un matériau ternaire à base de GaAs) épitaxié sur le substrat en arséniure de gallium GAS, une couche réflectrice inférieure BBR, une couche active AL, une couche réflectrice supérieure TBR et un contact semiconducteur supérieur TSCC en arséniure de gallium.

De même que dans le procédé 100 dans une étape **F2** on déposer une deuxième couche diélectrique DL2 et une deuxième couche de résine RL2, on structurer la deuxième couche de résine RL2 de manière à former un deuxième masque M2 présentant des deuxièmes ouvertures Op2 au-dessus de chaque empilement, on grave la deuxième couche diélectrique, on grave le contact semiconducteur supérieur et on enlève la deuxième couche de résine.

Dans une étape **G2** on enlève les première et deuxième couches diélectriques par gravure humide, de manière à obtenir des empilements disposés sur le substrat en arséniure de gallium GAS. On utilise ici une gravure humide pour ne pas dégrader le graphène restant de chaque côté de l'empilement.

Dans une étape **H2** on dépose le contact métallique inférieur BMC sur la couche de graphène restante de chaque côté des empilements, on déposer une couche isolante IL autour des empilements et on dépose un contact métallique supérieur TMC en contact avec le contact semiconducteur supérieur TSCC. C'est également à ce stade que l'on réalise la ou les pistes inférieures et les pistes supérieures pour la connectique.

Dans une étape **I2** on retire le substrat en arséniure de gallium GAS à l'aide de la couche isolante de substrat ILS, préférentiellement par clivage mécanique.

Dans une étape **J2** on retire la couche métallique ML et on dépose une deuxième couche de graphène GL2 sur la première couche de graphène GL1 et le contact semiconducteur inférieur, la première et la deuxième couche de graphène formant collectivement un substrat souple en graphène GS.

Cette deuxième couche de graphène assure la solidité et la flexibilité du substrat en graphène et l'injection du courant. Les empilements et les premier et deuxième contacts métallique associés sont configurés pour former des microlasers semi-conducteur à cavité verticale et émission par la surface µVL insérés dans la couche isolante, dénommés lasers élémentaires. Les lasers élémentaires sont disposés sur le substrat en graphène GS, et ici le substrat en graphène comprend des logements dans lesquels sont disposés les lasers élémentaires.

Le procédé 200 comprenant en outre une étape **K2** d'encapsulation du substrat et des lasers élémentaires avec un matériau biocompatible.

Ainsi dans ce procédé on grave le graphène jusqu'au GaAs, on fait croître le composant laser sur un substrat en GaAs GAS, puis on décolle le composant du substrat GAS à l'aide de la couche isolante de substrat.

## Revendications

1. Sonde médicale (MP) pour optogénétique comprenant :
- un substrat souple en matériau bidimensionnel conducteur (M2DS),
- une pluralité de microlasers à semi-conducteur III-V à cavité verticale et émission par la surface (µVL), dénommés lasers élémentaires, les lasers élémentaires étant disposés sur ledit substrat et intégrés dans une couche isolante (IL), les lasers élémentaires présentant une dimension maximale comprise entre 5 et 50 µm et comprenant :
∘ une couche active (AL) disposée entre une couche réflectrice inférieure (BBR) et une couche réflectrice supérieure (TBR),
∘ un contact semiconducteur inférieur (BSCC) disposé entre la couche réflectrice inférieure et le substrat et un contact métallique inférieur (BMC) disposé sur le substrat et connecté audit contact semiconducteur inférieur (BSCC) via ledit substrat (M2DS),
∘ un contact semiconducteur supérieur (TSCC) disposé sur la couche réflectrice supérieure (TBR), et un contact métallique supérieur (TMC) connecté audit contact semiconducteur supérieur,
∘ les contacts métalliques inférieurs des lasers élémentaires étant destinés à être connectés électriquement à un potentiel commun,
- une couche d'encapsulation biocompatible.

2. Sonde médicale (MP) selon la revendication précédente dans laquelle le matériau bidimensionnel est le graphène.

3. Sonde médicale (MP) selon la revendication 1 dans laquelle le matériau bidimensionnel est un dichalcogénure ou un trichalcogénure configuré pour être conducteur.

4. Sonde médicale selon l'une des revendications précédentes dans laquelle le substrat présente une forme de ruban sur une partie duquel lesdits lasers élémentaires de ladite pluralité sont disposés en ligne, les contacts métalliques inférieurs des lasers élémentaires étant connectés à une piste électrique inférieure (CBMT) commune audits lasers élémentaires de la ligne.

5. Sonde médicale selon l'une des revendications 1 à 3 dans laquelle les lasers élémentaires de ladite pluralité sont disposés selon une matrice, les contacts semiconducteurs inférieurs des lasers élémentaires d'une ligne de la matrice étant connectés à une piste électrique inférieure commune aux lasers élémentaires de ladite ligne de la matrice, les pistes électriques inférieure associées aux lignes étant connectées entre elles.

6. Sonde médicale selon l'une des revendications précédentes dans laquelle les contacts semiconducteurs inférieur et supérieur d'un laser élémentaire sont en nitrure de gallium (GaN) ou en un matériau ternaire comprenant du nitrure de gallium.

7. Sonde médicale selon la revendication 6 dans laquelle le matériau bidimensionnel est le graphène et le contact semiconducteur inférieur présente un axe cristallographique de croissance selon l'axe [1000].

8. Sonde médicale selon la revendication 6 dans laquelle le matériau bidimensionnel est un dichalcogénure choisi parmi WS₂, MoS₂, ReS₂ et le contact semiconducteur inférieur présente un axe cristallographique de croissance selon l'axe [100].

9. Sonde médicale selon l'une des revendications 1 à 5 dans laquelle les contacts semiconducteurs inférieur et supérieur d'un laser élémentaire sont en arséniure de gallium (GaAs) ou en un matériau ternaire comprenant de l'arséniure de gallium.

10. Sonde médicale selon la revendication précédente dans laquelle le matériau bidimensionnel est le graphène, et dans laquelle le substrat en graphène comprend des logements dans lesquels sont disposés les lasers élémentaires.

11. Sonde médicale selon l'une des revendications précédentes dans laquelle la couche active comprend des multipuits quantiques (MQW) ou des points quantiques (QD).

12. Sonde médicale selon l'une des revendications précédentes dans laquelle le substrat en matériau bidimensionnel et les lasers élémentaires forment une première structure (S1), la sonde comprenant au moins une deuxième structure (S2) empilée sur la première structure (S1), des lasers élémentaires des deux structures étant agencés de sorte que des lasers élémentaires (µVL2) de la deuxième structure n'occultent pas un faisceau émis (ELB1) par des lasers élémentaires (µVL1) de la première structure.

13. Sonde médicale selon la revendication précédente dans laquelle les lasers élémentaires dans la deuxième structure sont configurés pour émettre une longueur d'onde (λ2) différente d'une longueur d'onde d'émission (λ1) de la première structure.

14. Sonde médicale selon la revendication précédente dans laquelle une des deux structures est constituée de lasers élémentaires comprenant au moins une couche en nitrure de gallium ou un matériau ternaire comprenant du nitrure de gallium, et l'autre structure est constituée de lasers élémentaires comprenant au moins une couche en arséniure de gallium ou un matériau ternaire comprenant du nitrure de gallium.

15. Procédé (100) de fabrication d'une sonde médicale pour optogénétique comprenant les étapes consistant à :
**A1** disposer d'un premier substrat initial (IS1) comprenant un substrat semiconducteur (SS), une couche isolante dite de substrat (ILS), une couche métallique (ML) et une couche de graphène (GS) disposé sur la couche métallique,
**B1** déposer sur le premier substrat initial une première couche diélectrique (DL1) et une première couche de résine (RL1), structurer la première couche de résine de manière à former un premier masque (M1) présentant des premières ouvertures (Op1), les premières ouvertures présentant une dimension maximale comprise entre 5 et 50 µm,
**C1** graver la première couche diélectrique jusqu'à la couche de graphène par gravure humide, de manière à mettre à nu la couche de graphène dans les premières ouvertures,
**D1** enlever la première couche de résine,
**E1** réaliser par épitaxie, dans lesdites premières ouvertures (Op1), un empilement de matériaux semiconducteurs, l'empilement comprenant un contact semiconducteur inférieur (BSCC) en nitrure de gallium épitaxié sur la couche de graphène, une couche réflectrice inférieure (BBR), une couche active (AL), une couche réflectrice supérieure (TBR) et un contact semiconducteur supérieur (TSCC) en nitrure de gallium,
**F1** déposer une deuxième couche diélectrique (DL2), structurer une deuxième couche de résine (RL2) de manière à former un deuxième masque (M2) présentant des deuxièmes ouvertures (Op2) au-dessus de chaque empilement, graver la deuxième couche diélectrique et le contact semiconducteur supérieur et enlever la deuxième couche de résine,
**G1** enlever les première et deuxième couches diélectriques par gravure humide, de manière à obtenir des empilements disposés sur le premier substrat initial,
**H1** déposer un contact métallique inférieur (BMC) sur la couche de graphène, déposer une couche isolante autour des empilements et déposer un contact métallique supérieur (TMC) connecté avec le contact semiconducteur supérieur (TSCC),
**I1** retirer le substrat semiconducteur (SS) à l'aide de la couche isolante de substrat (ILS),
**J1** retirer la couche métallique (ML),
les empilements et les premier et deuxième contacts métallique associés étant configurés pour former des microlasers semi-conducteur à cavité verticale et émission par la surface (µVL) insérés dans la couche isolante, dénommés lasers élémentaires, la couche de graphène formant un substrat souple (GS), les lasers élémentaires étant disposés sur ledit substrat souple,
le procédé comprenant en outre une étape **K1** d'encapsulation du substrat et des lasers élémentaires avec un matériau biocompatible.

16. Procédé selon la revendication précédente dans lequel lors de l'étape **E1** la croissance du contact semiconducteur inférieur en nitrure de gallium par épitaxie sur le substrat en graphène s'opère selon une direction [1000].

17. Procédé selon l'une des revendications 15 ou 16 dans lequel à l'étape **C1** la gravure humide de la première couche diélectrique jusqu'à la couche de graphène est de type BOE pour « Buffered Oxide Etching ».

18. Procédé (200) de fabrication d'une sonde médicale pour optogénétique comprenant les étapes consistant à :
**A2** disposer d'un deuxième substrat initial (IS2) comprenant un substrat en arséniure de gallium (GAS), une couche isolante dite de substrat (ILS), une couche métallique (ML) et une première couche de graphène (GL1) disposée sur la couche métallique,
**B2** déposer sur le deuxième substrat initial une première couche diélectrique (DL1) et une première couche de résine (RL1), structurer la première couche de résine de manière à former un premier masque (M1) présentant des premières ouvertures (Op1), les premières ouvertures présentant une dimension maximale comprise entre 5 et 50 µm,
**C2** graver la première couche diélectrique jusqu'à la couche de graphène,
**D2** enlever la première couche de résine, graver la couche de graphène, graver la couche métallique et graver la couche isolante de substrat, de manière à mettre à nu le substrat en arséniure de gallium dans les premières ouvertures,
**E2** réaliser par épitaxie, dans lesdites premières ouvertures (Op1), un empilement de matériaux semiconducteurs comprenant un contact semiconducteur inférieur (BSCC) en arséniure de gallium épitaxié sur le substrat en arséniure de gallium, une couche réflectrice inférieure (BBR), une couche active (AL), une couche réflectrice supérieure (TBR) et un contact semiconducteur supérieur (TSCC) en arséniure de gallium,
**F2** déposer une deuxième couche diélectrique (DL2) et une deuxième couche de résine (RL2), structurer la deuxième couche de résine (RL2) de manière à former un deuxième masque (M2) présentant des deuxièmes ouvertures (Op2) au-dessus de chaque empilement, graver la deuxième couche diélectrique, graver le contact semiconducteur supérieur, et enlever la deuxième couche de résine,
**G2** enlever les première et deuxième couches diélectriques par gravure humide, de manière à obtenir des empilements disposés sur le substrat en arséniure de gallium,
**H2** déposer un contact métallique inférieur (BMC) sur la couche de graphène restante de chaque côté des empilements, déposer une couche isolante autour des empilements et déposer un contact métallique supérieur (TMC) en contact avec le contact semiconducteur supérieur (TSCC),
**12** retirer le substrat en arséniure de gallium (GAS) à l'aide de la couche isolante de substrat (ILS),
**J2** retirer la couche métallique (ML), et déposer une deuxième couche de graphène (GL2) sur la première couche de graphène et le contact semiconducteur inférieur, la première et la deuxième couche de graphène formant collectivement un substrat souple en graphène (GS),
les empilements et les premier et deuxième contacts métallique associés étant configurés pour former des microlasers semi-conducteur à cavité verticale et émission par la surface (µVL) insérés dans la couche isolante, dénommés lasers élémentaires, les lasers élémentaires étant disposés sur ledit substrat en graphène, le substrat en graphène comprenant des logements dans lesquels sont disposés les lasers élémentaires,
le procédé comprenant en outre une étape **K2** d'encapsulation du substrat et des lasers élémentaires avec un matériau biocompatible.
